# FASCICULE DE BREVET EUROPEEN

(11) **EP 0 598 765 B1**
(45) Date de publication et mention de la délivrance du brevet: **25.10.1995**
(21) Numéro de dépôt: 92916431.7
(22) Date de dépôt: 10.07.1992
(51) Int. Cl.: C07D 471/04

(54) **DERIVE DE L'AMINO-2 NAPHTYRIDINE, SA PREPARATION ET SON EMPLOI**
2-AMINONAPHTHYRIDIN-DERIVAT, SEINE HERSTELLUNG UND VERWENDUNG
2-AMINONAPHTHYRIDINE DERIVATIVE, ITS PREPARATION AND USE

(30) Priorité: 12.07.1991 FR 9108827
(43) Date de publication de la demande: 01.06.1994
(73) Titulaire: RHONE-POULENC RORER S.A., 92160 Antony (FR)
(72) Inventeur: DAVID-COMTE, Marie-Thérèse, 91360 Villemoisson Sur Orge (FR); ROUSSEL, Gérard, F-91450 Soisy-sur-Seine (FR)
(74) Mandataire: Pilard, Jacques
(86) Numéro de dépôt international: FR9200669
(87) Numéro de publication internationale: WO9301190

(56) Documents cités:
- FR-A- 2 313 060
- FR-A- 2 321 290
- US-A- 4 960 779

## Description

La présente invention concerne un nouveau dérivé de l'amino-2 naphtyridine de formule : 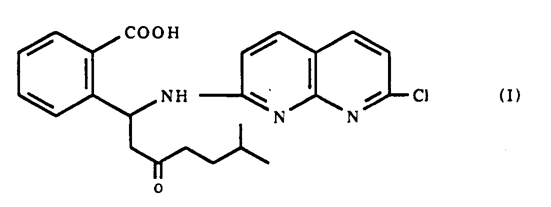
sa préparation et son emploi pour la préparation des isomères optiques du produit de formule : 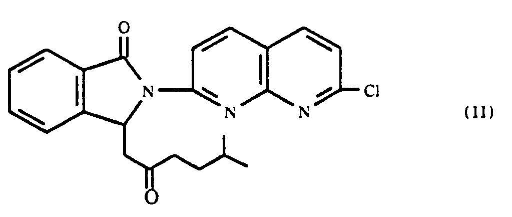
Le produit de formule (II) et des produits analogues qui présentent des propriétés anxiolytiques, hypnotiques, anticonvulsivantes, antiépileptiques et myorelaxantes remarquables font l'objet du brevet américain US 4 960 779. Il a été montré que, dans le produit de formule (II), l'entité active ou eutomère est l'isomère dextrogyre (+).

Selon le brevet américain US 4 960 779, la séparation des isomères optiques du produit de formule (II) peut être effectuée par chromatographie sur phase chirale. Cependant, l'application industrielle de ce procédé n'est pas toujours de réalisation commode.

Il a maintenant été trouvé, et c'est ce qui fait l'objet de la présente invention, que le produit de formule (I) qui possède un atome de carbone asymétrique et des fonctions acide et basique est particulièrement intéressant pour préparer l'isomère dextrogyre du produit de formule (II) par formation d'un sel avec une base chirale suivie de la cyclisation de l'isomère dextrogyre du produit de formule (I) en isomère dextrogyre du produit de formule (II).

Selon la présente invention, le produit de formule (I) peut être obtenu par ouverture du cycle pyrrolinone d'un produit de formule (II) racémique en milieu basique.

Généralement, l'ouverture du cycle pyrrolinone est effectuée au moyen d'une base minérale à une température comprise entre 0 et 50°C et, de préférence, comprise entre 0 et 30°C.

Généralement, le procédé est mis en oeuvre en agitant une solution hydro-organique du produit de formule (II) en présence d'un excès de base minérale choisie parmie les hydroxydes et les carbonates ou bicarbonates de métaux alcalins ou alcalino-tereux. Il est particulièrement avantageux d'utiliser la soude comme base minérale et d'opérer dans un mélange eau-pyridine. Il est aussi possible d'effectuer la réaction en utilisant un mélange eau-dioxanne comme solvant.

Selon l'invention, le nouveau produit de formule (I) peut aussi être obtenu par action d'une base minérale sur le produit de formule : 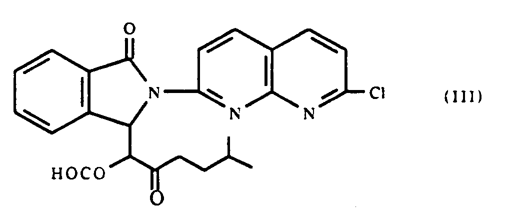
Généralement, on fait agir au moins deux équivalents de la base minérale choisie, de préférence, parmi la soude, la potasse, le carbonate de sodium ou le carbonate de potassium en opérant dans l'eau ou dans un milieu hydro-organique à une température comprise entre 0 et 50°C, de préférence, entre 0 et 30°C. Comme milieu hydro-organique, on utilise de préférence un mélange pyridine-eau.

Le produit de formule (III) peut être obtenu par hydrolyse en milieu acide d'un produit de formule générale : 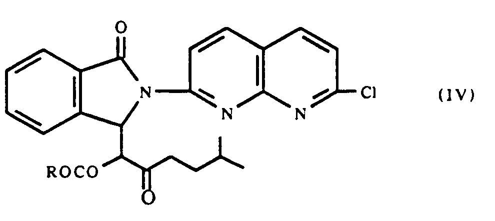
dans laquelle R représente un radical alkyle contenant 1 à 10 atomes de carbone en chaîne droite ou ramifiée.

Généralement, l'hydrolyse est effectuée au moyen d'un acide minéral fort tel que l'acide sulfurique concentré en opérant à une température comprise entre 0 et 50°C, de préférence voisine de 20°C.

Les produits de formule (III) et (IV) peuvent être obtenus dans les conditions décrites dans le brevet américain US 4 960 779.

Le nouveau produit de formule (I) peut être purifié par les méthodes connues telles que par des extractions successives en milieu acide et basique.

Le nouveau produit de formule (I) peut être transformé en sel d'addition avec les acides (acides chlorhydrique, méthanesulfonique, oxalique, maléique, fumarique) ou en sel avec les bases minérales (soude, potasse) ou organiques.

Pour préparer l'eutomère du produit de formule (II) il est particulièrement avantageux d'effectuer la succession des opérations suivantes :
1) formation d'un sel avec une base chirale ou un acide chiral,
2) précipitation d'un des isomères optiques,
3) libération de l'isomère optique dextrogyre du produit de formule (I), soit à partir du sel précipité soit à partir des eaux-mères de filtration du sel précipité après formation éventuelle d'un autre sel avec une base chirale ou un acide chiral appropriés, puis
4) cyclisation de l'isomère optique dextrogyre du produit de formule (I) en isomère dextrogyre du produit de formule (II) dans des conditions non racémisantes.

Ainsi, il est possible de former un sel du produit de formule (I) racémique avec la (+)-éphédrine en opérant dans un solvant organique approprié tel que l'éthanol. Le sel du produit de formule (I) dextrogyre et de la (+)-éphédrine précipite. L'isomère dextrogyre du produit de formule (I) qui est déplacé de son sel au moyen d'un acide fort est cyclisé en eutomère du produit de formule (II) au moyen de chlorure de thionyle en opérant éventuellement en présence d'un agent de condensation tel que l'imidazole ou la pyridine dans un solvant organique tel que le chlorure de méthylène.

Il n'est pas nécessaire d'isoler l'isomère dextrogyre du produit de formule (I) préalablement à la cyclisation en produit de formule (II) dextrogyre.

Il est également possible de préparer un sel du produit de formule (I) avec la cinchonine dans un solvant approprié tel que l'éthanol. Le sel du produit de formule (I) lévogyre avec la cinchonine précipite. Après déplacement de son sel avec la cinchonine, l'isomère dextrogyre du produit de formule (I), qui se trouve majoritairement dans les eaux-mères de filtration du sel lévogyre, est transformé en sel insoluble avec la cinchonidine. L'isomère dextrogyre du produit de formule (I), qui est déplacé de son sel avec la cinchonidine, est cyclisé en eutomère du produit de formule (II) dans les conditions décrites précédemment.

Les exemples suivants illustrent la présente invention.

### EXEMPLE 1

Dans un réacteur agité de 2 litres, on introduit à une température voisine de 20°C, 1400 cm3 de dioxanne et 20 g de (±)-(chloro-7 naphtyridine-1,8 yl-2)-2 (méthyl-5 oxo-2 hexyl)-3 isoindolinone-1. On additionne en 5 minutes 244 cm3 d'une solution aqueuse de soude N. On laisse réagir pendant 4 jours à une température inférieure à 30°C.

Le dioxanne est éliminé par distillation sous pression réduite (40 mm de mercure ; 5,3 kPa) à une température inférieure à 30°C. Pendant la distillation, on ajoute 100 cm3 d'eau distillée.

On élimine un produit insoluble par filtration à 20°C. Ce produit est lavé par 3 fois 50 cm3 d'eau distillée et est éliminé. Les phases aqueuses réunies sont acidifiées par addition, en 3 heures, de 48 cm3 d'acide chlorhydrique 5N à une température de 20°C. Le pH de la suspension est alors voisin de 3,5.

Après filtration de la suspension, le précipité est lavé par 6 fois 100 cm3 d'eau distillée puis séché sous pression réduite (15 mm de mercure ; 2,0 kPa) à 60°C pendant 16 heures.

On obtient ainsi 14,3 g d'acide (±)-{[(chloro-7 naphtyridine-1,8 yl-2) amino]-1 méthyl-6 oxo-3 heptyl}-2 benzoïque sous forme d'un produit blanc dont le temps de rétention est de 4,8 minutes par chromatographie liquide à haute performance en utilisant une colonne de 25 cm de longueur et de 0,46 cm de diamètre avec comme phase stationnaire du "Lichrospher O.D.S. 5 µm" et comme phase mobile un mélange de 200 cm3 de tampon phosphate pH 3 25 mM, de 560 cm3 d'acétonitrile et de 240 cm3 de méthanol au débit de 0,8 cm3/minute.

La (±)-(chloro-7 naphtyridine-1,8 yl-2)-2 (méthyl-5 oxo-2 hexyl)-3 isoindolinone-1 peut être préparé selon la méthode décrite dans le brevet américain US 4 960 779.

### EXEMPLE 2

Dans un réacteur agité de 1 litre, on introduit à une température voisine de 20°C, 20 g de (±)-(chloro-7 naphtyridine-1,8 yl-2)-2 (méthyl-5 oxo-2 hexyl)-3 isoindolinone-1, 400 cm3 de pyridine et 60 cm3 d'une solution aqueuse de soude 2N. On laisse réagir pendant 23 heures puis on distille la pyridine sous pression réduite (15 mm de mercure ; 2,0 kPa) à une température inférieure à 20°C. On ajoute 500 cm3 d'eau distillée. Un insoluble est séparé par filtration. La phase aqueuse est acidifiée à pH = 3,8 par addition de 40 cm3 d'acide chlorhydrique 4N. La suspension est filtrée, le précipité est lavé par 5 fois 140 cm3 d'eau distillée puis séché pendant 16 heures sous pression réduite (15 mm de mercure ; 2,0 kPa) à 60°C.

On obtient ainsi 19,2 g d'acide (±)-{[(chloro-7 naphtyridine-1,8 yl-2) amino]-1 méthyl-6 oxo-3 heptyl}-2 benzoïque sous forme d'un produit blanc dont le temps de rétention par chromatographie liquide à haute performance est de 4,8 minutes dans les conditions décrites à l'exemple 1.

### EXEMPLE 3

Une suspension de 30 mg d'acide (±)-[(chloro-7 naphtyridine-1,8 yl-2)-2 oxo-3 isoindolinyl-1]-2 méthyl-6 oxo-3 heptanoïque dans 4,7 cm3 d'eau distillée et 1,32 cm3 d'une solution aqueuse de soude 0,1N est agitée à une température voisine de 20°C pendant 72 heures. On élimine un produit insoluble par filtration et le filtrat est acidifié jusqu'à pH = 2 par addition d'une solution aqueuse d'acide chlorhydrique 0,1N. Le précipité obtenu est séparé par filtration, lavé à l'eau et séché à l'air. On obtient ainsi 10 mg d'acide (±)-{[(chloro-7 naphtyridine-1,8 yl-2) amino]-1 méthyl-6 oxo-3 heptyl}-2 benzoïque dont les caractéristiques sont identiques à celles du produit de l'exemple 1.

L'acide (±)-[(chloro-7 naphtyridine-1,8 yl-2)-2 oxo-3 isoindolinyl-1]-2 méthyl-6 oxo-3 heptanoïque peut être préparé de la façon suivante :

Une solution de 23 g de (±)-[(chloro-7 naphtyridine-1,8 yl-2)-2 oxo-3 isoindolinyl-1]-2 méthyl-6 oxo-3 heptanoate d'éthyle dans 235 cm3 d'acide sulfurique à 98 %, est agitée pendant 20 heures à une température voisine de 20°C puis versée dans 1,5 kg de glace. Le précipité obtenu est séparé par filtration, lavé à l'eau jusqu'à pH = 6 et séché à l'air. Le solide obtenu est repris par 3,8 litres d'eau distillée et 480 cm3 d'une solution aqueuse de soude 0,1N. Le produit insoluble est séparé par filtration, le filtrat est acidifié jusqu'à pH = 3 par addition d'une solution aqueuse d'acide chlorhydrique 0,1N. Le précipité obtenu est séparé par filtration, lavé à l'eau distillée puis à l'oxyde d'isopropyle et séché à 20°C sous pression réduite (0,07 kPa). On obtient ainsi 9,2 g d'acide (±)-[(chloro-7 naphtyridine-1,8 yl-2)-2 oxo-3 isoindolinyl-1]-2 méthyl-6 oxo-3 heptanoïque fondant à 176°C.

Le (±)-[(chloro-7 naphryridine-1,8 yl-2)-2 oxo-3 isoindolinyl-1]-2 méthyl-6 oxo-3 heptanoate d'éthyle peut être obtenu par la méthode décrite dans le brevet américain US 4 960 779.

### EXEMPLE 4

1) Dans un réacteur agité de 2 litres, on introduit 1450 cm3 d'éthanol à 95 % (v/v), 100 g de cinchonine et 145 g d'acide (±)-{[(chloro-7 naphtyridine-1,8 yl-2) amino]-1 méthyl-6 oxo-3 heptyl}-2 benzoïque. La suspension est chauffée à 40°C puis refroidie, en 3 heures 30 minutes, à 10°C. La suspension obtenue est filtrée. Le précipité est lavé par 2 fois 50 cm3 d'éthanol à 10°C puis séché à 60°C pendant 16 heures sous pression réduite (15 mm de mercure ; 2,0 kPa). On obtient ainsi 99,3 g de sel de cinchonine et d'acide (-)-{[(chloro-7 naphtyridine-1,8 yl-2) amino]-1 méthyl-6 oxo-3 heptyl}-2 benzoïque sous forme d'un produit blanc dont les caractéristiques sont les suivantes :
   - pouvoir rotatoire : [α]²⁰_{D} = +192,6° (c = 1 ; chlorure de méthylène)
   - titre énantiomérique : 98,5 %
   Dans un réacteur agité de 2 litres, on introduit 250 cm3 de N-méthylpyrrolidone et 50 g du sel obtenu précédemment. On ajoute, en 30 minutes, en maintenant la température à 20°C, 90 cm3 d'acide chlorhydrique N. On laisse pendant 1 heure à cette température puis on ajoute, en 1 heure, 660 cm3 d'eau distillée. La suspension obtenue est filtrée. Le précipité est lavé par 5 fois 100 cm3 d'eau puis séché à 60°C pendant 16 heures sous pression réduite (15 mm de mercure ; 2,0 kPa).
   On obtient ainsi 28,6 g d'acide (-)-{[(chloro-7 naphtyridine-1,8 yl-2) amino]-1 méthyl-6 oxo-3 heptyl}-2 benzoïque sous forme d'un produit blanc dont les caractéristiques sont les suivantes :
   - pouvoir rotatoire : [α]²⁰_{D} = -227,4° (c = 1 ; chlorure de méthylène)
   - titre énantiomérique : 99,6 %.

   Dans un réacteur agité de 1 litre, on introduit à une température de 20°C, 400 cm3 de chlorure de méthylène, 20 g du produit obtenu précédemment et 21,8 g d'imidazole. A l'aide d'une seringue, on ajoute, en 10 minutes, 7 cm3 de chlorure de thionyle. La suspension est chauffée au reflux pendant 30 minutes puis est refroidie à 20°C et lavée par 2 fois 200 cm3 d'eau distillée. La solution lavée est concentrée à la moitié de son volume puis on ajoute 450 cm3 d'éthanol absolu. La distillation sous pression atmosphérique est poursuivie jusqu'à ce que la température des vapeurs soit de 78°C. On ajoute 1 g de noir décolorant puis maintient pendant 1 heure à 70°C. La suspension est filtrée. Le précipité est lavé par 50 cm3 d'éthanol à 75°C. Le filtrat et le lavage sont réunis. Après refroidissement en 2 heures à 15°C, la suspension est filtrée. Le précipité est lavé par 3 fois 35 cm3 d'éthanol puis séché à 60°C pendant 16 heures sous pression réduite (15 mm de mercure ; 2,0 kPa).
   On obtient ainsi 16,7 g de (-)-(chloro-7 naphryridine-1,8 yl)-2 (méthyl-5 oxo-2 hexyl)-3 isoindolinone-1 sous forme d'un produit cotonneux blanc dont les caractéristiques sont les suivantes :
   - pouvoir rotatoire : [α]²⁰_{D} = -132° (c = 1 ; chlorure de méthylène)
   - titre énantiomérique : 100 %
2) Dans un réacteur de 2 litres, on introduit 1274,3 g de liqueurs éthanoliques (correspondant au filtrat de sel de cinchonine obtenu précédemment par addition du lavage éthanolique). A 20°C, on ajoute 260 cm3 d'une solution aqueuse d'acide chlorhydrique N. Après 15 minutes d'agitation, on ajoute 650 cm3 d'eau distillée. La solution est concentrée sous pression réduite (25 mm de mercure ; 3,3 kPa) à une température inférieure à 30°C pour éliminer l'éthanol. La suspension est ensuite filtrée. Le précipité est lavé par 6 fois 100 cm3 d'eau puis séché à 60°C pendant 16 heures sous pression réduite (15 mm de mercure ; 2,0 kPa).

On obtient ainsi 79,6 g d'un produit blanc constitué majoritairement de l'isomère dextrogyre de l'acide {[(chloro-7 naphryridine-1,8 yl-2) amino]-1 méthyl-6 oxo-3 heptyl}-2 benzoïque dont les caractéristiques sont les suivantes :
- pouvoir rotatoire : [α]²⁰_{D} = +160° (c = 1 ; chlorure de méthylène)
- titre énantiomérique : 73,2 %
Dans un réacteur de 1 litre, on introduit 78,5 g du produit obtenu précédemment, 54,3 g de cinchonidine et 700 cm3 d'éthanol à 95 % (v/v). La solution est chauffée à reflux puis refroidie en 3 heures à une température de 10°C. Un produit cristallise. La suspension est filtrée. Le précipité est lavé avec 2 fois 50 cm3 d'éthanol à 95 % puis séché à 60°C pendant 16 heures sous pression réduite (15 mm de mercure ; 2,0 kPa).

On obtient ainsi 92,8 g de sel de cinchonidine de l'acide (+)-{[(chloro-7 naphtyridine-1,8 yl-2) amino]-1 méthyl-6 oxo-3 heptyl}-2 benzoïque sous forme d'un produit blanc dont les caractéristiques sont les suivantes :
- pouvoir rotatoire : [α]²⁰_{D} = -137,7° (c = 1 ; chlorure de méthylène)
- titre énantiomérique : 100 %
Dans un réacteur de 1 litre, on dissout 50 g du sel de cinchonidine obtenu précédemment dans 250 cm3 de N-méthylpyrrolidone. On ajoute, en 30 minutes, 90 cm3 d'acide chlorhydrique N en maintenant la température inférieure à 20°C. Après 15 minutes d'agitation à 20°C, on ajoute, en 1 heure, 600 cm3 d'eau distillée. La suspension obtenue est filtrée. Le précipité obtenu est lavé par 5 fois 100 cm3 d'eau distillée puis séché à 60°C pendant 16 heures sous pression réduite (15 mm de mercure ; 2,0 kPa).

On obtient ainsi 29,7 g d'acide (+)-{[(chloro-7 naphtyridine-1,8 yl-2) amino]-1 méthyl-6 oxo-3 heptyl}-2 benzoïque sous forme d'un produit blanc dont les caractéristiques sont les suivantes :
- pouvoir rotatoire : [α]²⁰_{D} = +222,8° (c = 1 ; chlorure de méthylène)
- titre énantiomérique : 100 %
Dans un réacteur de 1 litre, on dissout 20 g de l'acide dextrogyre obtenu précédemment et 21,8 g d'imidazole dans 400 cm3 de chlorure de méthylène. A une température de 20°C, on introduit, à l'aide d'une seringue, 7 cm3 de chlorure de thionyle. La suspension est chauffée au reflux pendant 30 minutes, est ensuite refroidie à 20°C puis lavée 2 fois par 200 cm3 d'eau distillée. La solution est concentrée, sous pression atmosphérique, à la moitié de son volume puis on ajoute 450 cm3 d'éthanol absolu. La distillation du chlorure de méthylène est poursuivie jusqu'à ce que la température des vapeurs atteigne 78°C. On ajoute alors 1 g de noir décolorant puis laisse une heure à 78°C. La suspension est filtrée. Le précipité est lavé par 50 cm3 d'éthanol absolu à 75°C. Le filtrat et les lavages sont réunis puis refroidis à 15°C en 2 heures. La suspension est filtrée. Le précipité est lavé par 3 fois 35 cm3 d'éthanol absolu à 15°C puis séché à 60°C pendant 16 heures sous pression réduite (15 mm de mercure ; 2,0 kPa). On obtient ainsi 16,8 g de (+)-(chloro-7 naphtyridine-1,8 yl)-2 (méthyl-5 oxo-2 hexyl)-3 isoindolinone-1 sous forme d'un produit cotonneux blanc dont les caractéristiques sont les suivantes :
- pouvoir rotatoire : [α]²⁰_{D} = +132° (c = 1 ; chlorure de méthylène)
- titre énantiomérique : 98,8 %.

### EXEMPLE 5

Dans un réacteur de 2 litres, on introduit, à une température voisine de 20°C, 250 g d'acide (±)-{[(chloro-7 naphtyridine-1,8 yl-2) amino]-1 méthyl-6 oxo-3 heptyl}-2 benzoïque, 97 g de (+)-éphédrine et 875 cm3 d'éthanol à 95 % (v/v). Après dissolution de la suspension à 40°C, on refroidit le mélange réactionnel au voisinage de 2°C. Le précipité obtenu est séparé par filtration, lavé par 2 fois 125 cm3 d'éthanol à 95 % (v/v) à 2°C puis séché pendant 16 heures à 60°C sous pression réduite (15 mm de mercure ; 2,0 kPa). On obtient ainsi 156,6 g de sel de (+)-éphédrine et d'acide (+)-{[(chloro-7 naphtyridine-1,8 yl-2) amino]-1 méthyl-6 oxo-3 heptyl}-2 benzoïque sous forme d'un produit blanc dont les caractéristiques sont les suivantes :
- pouvoir rotatoire : [α]²⁰_{D} = -64° (c = 1 ; chlorure de méthylène)
- titre énantiomérique : 100 %.

Dans un ballon de 50 cm3 on introduit 2,75 g du sel obtenu précédemment et 5 cm3 de N-méthylpyrrolidone. La température étant maintenue à 20°C, on ajoute 1,2 cm3 d'acide chlorhydrique concentré puis ensuite, en 10 minutes, 15 cm3 d'eau distillée. La suspension obtenue est filtrée. Le précipité est lavé par 5 fois 10 cm3 d'eau distillée puis séché pendant 16 heures à 60°C sous pression réduite (15 mm de mercure ; 2,0 kPa).

On obtient ainsi 1,97 g d'acide (+)-{[(chloro-7 naphtyridine-1,8 yl-2) amino]-1 méthyl-6 oxo-3 heptyl}-2 benzoïque sous forme d'un produit blanc dont les caractéristiques sont les suivantes :
- pouvoir rotatoire : [α]²⁰_{D} = +222,8° (c = 1 ; chlorure de méthylène)
- titre énantiomérique : 100 %
Le produit ainsi obtenu est cyclisé en (+)-(chloro-7 naphtyridine-1,8 yl-2)-2 (méthyl-5 oxo-2 hexyl)-3 isoindolinone-1 dans les conditions décrites précédemment dans l'exemple 4.

### EXEMPLE 6

Dans un réacteur de 2,5 litres, on introduit 118,3 g de sel de (+)-éphédrine et d'acide (+)-{[(chloro-7 naphtyridine-1,8 yl-2) amino]-1 méthyl-6 oxo-3 heptyl}-2 benzoïque et 1700 cm3 de chlorure de méthylène. La solution organique est lavée, à 20°C, par 400 cm3 d'une solution aqueuse d'acide chlorhydrique 0,5N puis par 400 cm3 d'eau distillée. La phase organique est déshydratée par distillation azéotropique à 20°C sous pression réduite (250 mm de mercure ; 33,3 kPa). Le volume de la phase organique est ajusté à 1700 cm3 par addition de chlorure de méthylène sec puis on ajoute 95,2 g d'imidazole puis, en 10 minutes, 25 cm3 de chlorure de thionyle. La suspension est chauffée à 40°C pendant 30 minutes puis refroidie à 20°C et lavée par 2 fois 700 cm3 d'eau distillée. Le chlorure de méthylène est éliminé par distillation sous pression atmosphérique tout en ajoutant, à volume constant, 2500 cm3 d'éthanol absolu. Lorsque la température des vapeurs atteint 78°C, la distillation est arrêtée, puis on ajoute 4 g de noir décolorant en suspension dans 20 cm3 d'éthanol absolu. On laisse pendant 30 minutes à 78°C puis on filtre à chaud. Le noir décolorant est rincé par 200 cm3 d'éthanol à 77°C. Le lavage et le filtrat sont réunis puis refroidis, à la vitesse de 20°C/heure, à une température de 10°C. La suspension est filtrée. Le précipité est lavé par 3 fois 140 cm3 d'éthanol absolu à 10°C puis séché à 60°C pendant 16 heures sous pression réduite (15 mm de mercure ; 2,0 kPa). Le produit obtenu (68,9 g) légèrement jaune est recristallisé dans 1400 cm3 d'éthanol au reflux. Après refroidissement à 10°C, la suspension est filtrée. Le précipité est lavé par 3 fois 100 cm3 d'éthanol absolu à 10°C puis séché à 60°C pendant 16 heures sous pression réduite (15 mm de mercure ;2,0 kPa). On obtient ainsi 65,1 g de (+)-(chloro-7 naphtyridine-1,8 yl-2)-2 (méthyl-5 oxo-2 hexyl)-3 isoindolinone-1 sous forme d'un produit blanc cotonneux dont les caractéristiques sont les suivantes :
- pouvoir rotatoire : [α]²⁰_{D} = +132° (c = 1 ; chlorure de méthylène)
- titre énantiomérique : 100 %.

## Revendications

1. Dérivé de l'amino-2 naphtyridine de formule : 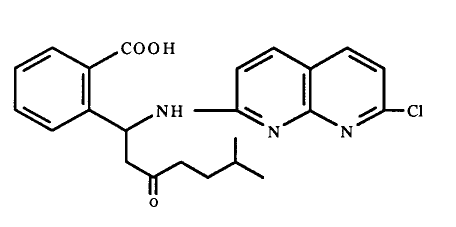 ses isomères optiques et ses sels.

2. Procédé de préparation du produit selon la revendication 1 caractérisé en ce que l'on fait réagir une base minérale sur un produit de formule : 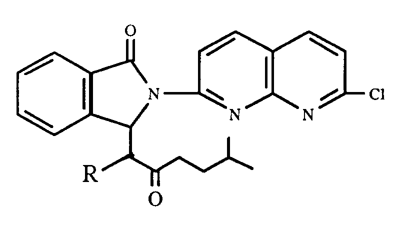 dans laquelle R représente un atome d'hydrogène ou un radical carboxy, puis isole le produit obtenu, le sépare éventuellement en ses isomères optiques et/ou le transforme éventuellement en sel.

3. Procédé de préparation de l'isomère dextrogyre du produit selon la revendication 1 caractérisé en ce que l'on forme un sel du produit racémique correspondant avec la (+)-éphédrine dans un solvant organique dans lequel le sel de l'isomère dextrogyre précipite, sépare ce dernier par filtration puis libère l'isomère dextrogyre de son sel au moyen d'un acide fort.

4. Procédé selon la revendication 3 caractérisé en ce que le solvant dans lequel le sel du produit racémique est précipité est l'éthanol.

5. Procédé selon la revendication 3 caractérisé en ce que la libération de l'isomère dextrogyre du produit selon la revendication 1 est effectué au moyen d'acide chlorhydrique.

6. Procédé de préparation de l'isomère dextrogyre du produit selon la revendication 1 caractérisé en ce que l'on forme un sel du produit racémique correspondant avec la cinchonine dans un solvant dans lequel le sel de l'isomère lévogyre précipite, sépare ce dernier par filtration, déplace le produit enrichi en isomère dextrogyre qui se trouve dans les eaux-mères de filtration de son sel avec la cinchonine, précipite le sel de l'isomère dextrogyre pur avec la cinchonidine, le sépare par filtration et enfin libère l'isomère dextrogyre de son sel avec la cinchonidine.

7. Procédé selon la revendication 6 caractérisé en ce que le sel du produit racémique avec la cinchonine est précipité dans l'éthanol.

8. Procédé selon la revendication 6 caractérisé en ce que le produit enrichi en isomère dextrogyre est séparé de son sel avec la cinchonine au moyen d'acide chlorhydrique.

9. Procédé selon la revendication 6 caractérisé en ce que le sel du produit enrichi en isomère dextrogyre avec la cinchonidine est précipité dans l'éthanol.

10. Procédé selon la revendication 6 caractérisé en ce que l'isomère dextrogyre pur est déplacé de son sel avec la cinchonidine au moyen d'acide chlorhydrique.

11. Procédé de préparation de l'isomère dextrogyre du produit de formule : 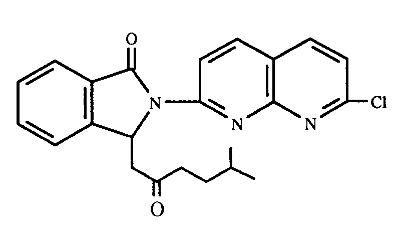 caractérisé en ce que l'on cyclise l'isomère dextrogyre du produit selon la revendication 1 au moyen de chlorure de thionyle en présence d'un agent de condensation dans un solvant organique, et isole le produit obtenu.

12. Procédé selon la revendication 11 caractérisé en ce que l'agent de condensation est l'imidazole ou la pyridine.

13. Procédé selon l'une des revendications 11 ou 12 caractérisé en ce que l'on opère dans le chlorure de méthylène.

## Claims

1. 2-Aminonaphthyridine derivative of formula: 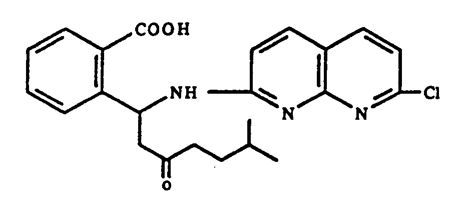 its optical isomers and its salts.

2. Process for the preparation of the product according to claim 1, characterized in that an inorganic base is reacted with a product of formula: 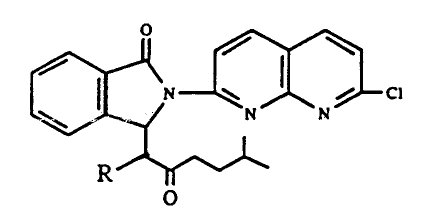 in which R represents a hydrogen atom or a carboxyl radical, the product obtained is then isolated, optionally separated into its optical isomers and/or optionally converted into a salt.

3. Process for the preparation of the dextrorotatory isomer of the product according to claim 1, characterized in that a salt of the corresponding racemic product with (+)-ephedrine is formed in an organic solvent in which the salt of the dextrorotatory isomer precipitates, the latter is separated by filtration and the dextrorotatory isomer is then released from its salt by means of a strong acid.

4. Process according to claim 3, characterized in that the solvent in which the salt of the racemic product is precipitated is ethanol.

5. Process according to claim 3, characterized in that the release of the dextrorotatory isomer of the product according to claim 1 is carried out by means of hydrochloric acid.

6. Process for the preparation of the dextrorotatory isomer of the product according to claim 1, characterized in that a salt of the corresponding racemic product with cinchonine is formed in a solvent in which the salt of the laevorotatory isomer precipitates, the latter is separated by filtration, the product which is enriched in the dextrorotatory isomer, which is found in the filtration mother liquors, is displaced from its salt with cinchonine, the salt of the pure dextrorotatory isomer with cinchonidine is precipitated and is separated by filtration and, finally, the dextrorotatory isomer is released from its salt with cinchonidine.

7. Process according to claim 6, characterized in that the salt of the racemic product with cinchonine is precipitated from ethanol.

8. Process according to claim 6, characterized in that the product which is enriched in the dextrorotatory isomer is separated from its salt with cinchonine by means of hydrochloric acid.

9. Process according to claim 6, characterized in that the salt of the product which is enriched in the dextrorotatory isomer with cinchonidine is precipitated from ethanol.

10. Process according to claim 6, characterized in that the pure dextrorotatory isomer is displaced from its salt with cinchonidine by means of hydrochloric acid.

11. Process for the preparation of the dextrorotatory isomer of the product of formula: 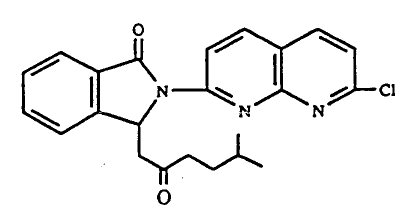 characterized in that the dextrorotatory isomer of the product according to claim 1 is cyclized by means of thionyl chloride in the presence of a condensation agent in an organic solvent and the product obtained is isolated.

12. Process according to claim 11, characterized in that the condensation agent is imidazole or pyridine.

13. Process according to either of claims 11 and 12, characterized in that the preparation is carried out in methylene chloride.

## Patentansprüche

1. 2-Amino-naphthyridin-Derivate der Formel 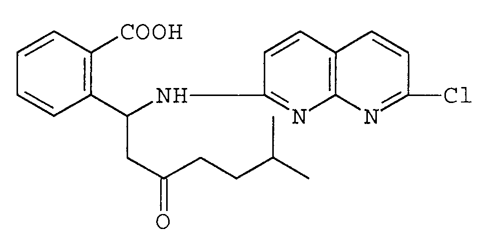 sowie ihre optischen Isomeren und ihre Salze.

2. Verfahren zur Herstellung des Produktes nach Anspruch 1, dadurch gekennzeichnet, daß man eine Mineralbase mit einem Produkt der Formel 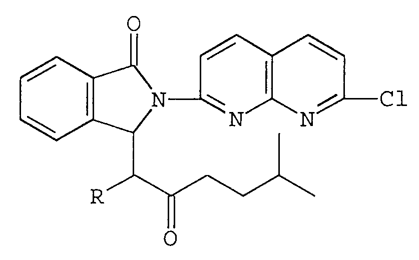 zur Reaktion bringt, in der R ein Wasserstoffatom oder einen Carboxyrest darstellt, und man anschließend das erhaltene Produkt isoliert, gegebenenfalls in seine optischen Isomeren trennt und/oder gegebenenfalls in ein Salz umwandelt.

3. Verfahren zur Herstellung des rechtsdrehenden Isomers des Produktes nach Anspruch 1, dadurch gekennzeichnet, daß man ein Salz des entsprechenden racemischen Produktes mit (+)-Ephedrin in einem organischen Lösungsmittel bildet, in dem das Salz des rechtsdrehenden Isomers ausfällt, dieses letztere mittels Filtration abtrennt und anschließend das rechtsdrehende Isomer mit Hilfe einer starken Säure aus seinem Salz freisetzt.

4. Verfahren nach Anspruch 3, dadurch gekennzeichnet, daß das Lösungsmittel, in dem das racemische Produkt gefällt wird, Ethanol ist.

5. Verfahren nach Anspruch 3, dadurch gekennzeichnet, daß die Freisetzung des rechtsdrehenden Isomers des Produktes nach Anspruch 1 mit Hilfe von Chlorwasserstoffsäure durchgeführt wird.

6. Verfahren zur Herstellung des rechtsdrehenden Isomers des Produktes nach Anspruch 1, dadurch gekennzeichnet, daß man ein Salz des entsprechenden racemischen Produktes mit Cinchonin in einem Lösungsmittel bildet, in dem das Salz des linksdrehenden Isomers ausfällt, dieses letztere mittels Filtration abtrennt, das mit dem rechtsdrehenden Isomer angereicherte Produkt, das sich in den Mutterlaugen der Filtration befindet, aus seinem Cinchoninsalz verdrängt, das Salz des reinen rechtsdrehenden Isomers mit Cinchonidin ausfällt, es mittels Filtration abtrennt und schließlich das rechtsdrehende Isomer aus seinem Salz mit Cinchonidin freisetzt.

7. Verfahren nach Anspruch 6, dadurch gekennzeichnet, daß das Salz des racemischen Produktes mit Cinchonin in Ethanol ausgefällt wird.

8. Verfahren nach Anspruch 6, dadurch gekennzeichnet, daß das mit dem rechtsdrehenden Isomer angereicherte Produkt von seinem Salz mit Cinchonin mit Hilfe von Chlorwasserstoffsäure getrennt wird.

9. Verfahren nach Anspruch 6, dadurch gekennzeichnet, daß das mit dem rechtsdrehenden Isomer angereicherte Produkt mit Cinchonidin in Ethanol ausgefällt wird.

10. Verfahren nach Anspruch 6, dadurch gekennzeichnet, daß das reine rechtsdrehende Isomer aus seinem Salz mit Cinchonidin mit Hilfe von Chlorwasserstoffsäure verdrängt wird.

11. Verfahren zur Herstellung des rechtsdrehenden Isomers des Produktes der Formel 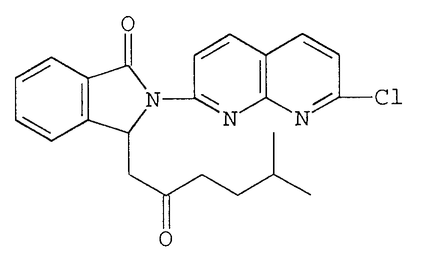 dadurch gekennzeichnet, daß man das rechtsdrehende Isomer des Produktes nach Anspruch 1 mit Hilfe von Thionylchlorid in Anwesenheit eines Kondensationsmittels in einem organischen Lösungsmittel cyclisiert und das erhaltene Produkt isoliert.

12. Verfahren nach Anspruch 11, dadurch gekennzeichnet, daß das Kondensationsmittel Imidazol oder Pyridin ist.

13. Verfahren nach einem der Ansprüche 11 oder 12, dadurch gekennzeichnet, daß man in Methylenchlorid arbeitet.
